## Europäisches Patentamt
## European Patent Office
## Office européen des brevets

(19)

(11) Veröffentlichungsnummer: **0 241 695**
**B1**

(12) # EUROPÄISCHE PATENTSCHRIFT

(45) Veröffentlichungstag der Patentschrift:
**16.08.90**

(51) Int. Cl.⁵: **A61N 1/32**

(21) Anmeldenummer: **87103180.3**

(22) Anmeldetag: **06.03.87**

(54) Interferenzstrom-Therapiegerät.

(30) Priorität: **19.03.86  DE 3609225**

(43) Veröffentlichungstag der Anmeldung:
**21.10.87 Patentblatt 87/43**

(45) Bekanntmachung des Hinweises auf die Patenterteilung:
**16.08.90 Patentblatt 90/33**

(84) Benannte Vertragsstaaten:
**AT CH DE GB LI NL**

(56) Entgegenhaltungen:
**DE-A- 3 344 831**

(73) Patentinhaber: **Siemens Aktiengesellschaft,
Wittelsbacherplatz 2, D-8000 München 2(DE)**

(72) Erfinder: **Helmreich, Klaus, Saalestrasse 15,
D-8520 Erlangen(DE)**

## Beschreibung

Die Erfindung bezieht sich auf ein Interferenzstrom-Therapiegerät gemäß den Merkmalen des Oberbegriffs des Patentanspruchs 1.

Es sind phasenmodulierende Geräte dieser Art bekannt (z.B. DE-PS 26 15 157), bei denen die erste Frequenz der ersten Wechselspannung beispielsweise $f_1 = 5$ kHz beträgt. Die zweite Frequenz der zweiten Wechselspannung beträgt $f_2 = f_1 + (\Delta f)_i$, wobei die Differenzfrequenz $(\Delta f)_i$ in fünf Stufen einstellbar ist, z.B. $(\Delta f)_1 = 0,1$ Hz bis 1 Hz (gewobbelt), $(\Delta f)_2 = 2,5$ Hz bis 25 Hz (gewobbelt), $(\Delta f)_3 = 10$ Hz, $(\Delta f)_4 = 50$ Hz und $(\Delta f)_5 = 200$ Hz. Die unterschiedlichen Differenzfrequenzen $(\Delta f)_i$ führen zu entsprechend unterschiedlichen Schwebungsfrequenzen im Patientenkörper, wenn der Reizstrom, der aufgrund Anlegung der ersten Wechselspannung an eine erste, am Patientenkörper angeordnete Reizstromelektrode erzeugt wird, dem Reizstrom überlagert wird, der aufgrund Anlegung der zweiten Wechselspannung an eine ebenfalls am Patientenkörper angeordnete zweite Reizstromelektrode erzeugt wird. In Fällen, in denen die Schwebungsfrequenz relativ niedrig ist, ergibt sich nun in Verbindung mit der Intensitätseinstellung das Problem, daß die Span-nungs- und damit auch die Stromintensität häufig zu hoch eingestellt wird, nämlich dann, wenn bei Änderung der Intensität die Schwebeschwingung gerade ein Tal durchläuft und anschließend langsam zum Maximum ansteigt. Eine zu hohe Stromintensität wird jedoch vom Patienten üblicherweise als schmerzhaft empfunden.

Aufgabe der vorliegenden Erfindung ist es, bei einem Interferenzstrom-Therapiegerät der eingangs genannten Art dafür zu sorgen, daß die Intensitätseinstellung auch bei niedrigen Schwebungsfrequenzen problemlos und für den Patienten immer schmerzlos erfolgt.

Die Aufgabe wird erfindungsgemäß mit den kennzeichnenden Merkmalen des Patentanspruchs 1 gelöst.

Beim Interferenzstrom-Therapiegerät nach der Erfindung ist die Frequenz der Schwebung während der Zeitdauer in der die Intensitätseinstellung geändert wird einschließlich des nachfolgenden Sicherheitsintervalls immer so hoch, daß mit Sicherheit wenigstens ein Maximum der Schwebung erreicht wird. Die Einstellung der Intensität kann also im Vergleich mit dem Schwebungsmaximum erfolgen. Ein unerwünscht hoher Intensitätswert kann dementsprechend nicht mehr irrtümlich eingestellt werden. Die Gefahr, daß der Patient durch schmerzhafte Empfindungen belästigt wird, ist nicht mehr gegeben.

In bevorzugter Ausgestaltung der Erfindung ist das Sicherheitsintervall wenigstens gleich oder größer als die Zeit, die der zu behandelnde Patient im Normalfall zur Reaktion auf die geänderte Intensität benötigt. Erfahrungsgemäß liegt die Reaktionszeit im Normalfall zwischen 1 bis 5 sec. Das Sicherheitsintervall soll dann also im Bereich 1 bis 5 sec, vorzugsweise bei 3 sec, liegen.

In weiterer vorteilhafter Ausgestaltung der Erfindung sollte der zweite Wechselspannungsgenerator sich selbsttätig für die Zeitdauer der Intensitätsänderung einschließlich des nachfolgenden Sicherheitsintervalls auf eine solche Frequenz der zweiten Wechselspannung einstellen, die sich von der Frequenz der ersten Wechselspannung um einen Differenzfrequenzwert $\geq 1$ Hz unterscheidet. Vorzugsweise sollte jedoch der Differenzfrequenzwert dem höchsten, in Abhängigkeit von der Frequenzauswahlvorrichtung vorgebbaren Differenzfrequenzwert (z.B. 200 Hz) entsprechen.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

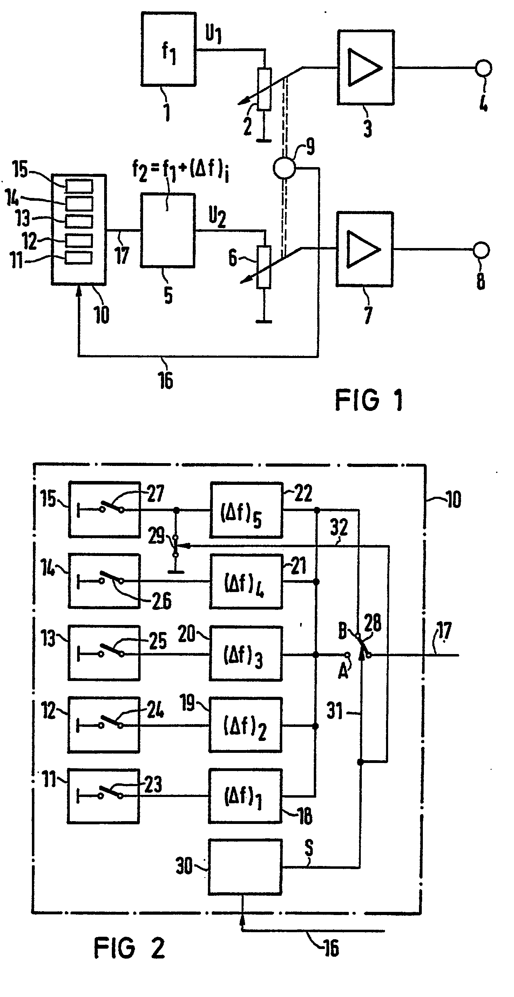

Es zeigen: Figur 1 die Erfindung im Prinzipschaltbild,

Figur 2 die Frequenzauswahlvorrichtung der Figur 1 in detaillierterer Darstellung,

Figur 3 den Zeitschalter der Figur 2 in detaillierter Darstellung und

Figur 4 eine Modifikation des Prinzipschaltbildes der Figur 1.

In der Figur 1 erzeugt ein erster Wechselspannungsgenerator 1 eine erste Wechselspannung $U_1$ mit einer ersten Frequenz von z.B. $f_1 = 5$ kHz. Die erste Wechselspannung $U_1$ wird über ein Widerstandspotentiometer 2 und einen Verstärker 3 einer ersten, an einen Patienten anlegbaren Reizstromelektrode 4 zugeführt. Ein zweiter Wechselspannungsgenerator 5 erzeugt eine zweite Wechselspannung $U_2$, deren Frequenz nach der Beziehung $f_2 = f_1 + (\Delta f)_i$ vorwählbar ist. Die zweite Wechselspannung $U_2$ wird über ein Widerstandspotentiometer 6 und einen Verstärker 7 einer zweiten Reizstromelektrode 8 zugeleitet. Die beiden Widerstandspotentiometer 2 und 3 sind synchron mittels Stellvorrichtung 9 (Drehknopf, Schieber od. dgl.) verstellbar. Die Bauelemente 2, 3, 6, 7 und 9 bilden zusammen eine Intensitätseinstellvorrichtung zum Einstellen der Intensität der ersten und zweiten Wechselspannung $U_1$ und $U_2$ und damit auch der dem Patienten über die Reizstromelektroden 4 und 8 zugeführten Reizströme.

Der Block 10 beinhaltet eine Frequenzanwahlvorrichtung für verschiedene Frequenzen $f_2 = f_1 + (\Delta f)_i$ der zweiten Wechselspannung $U_2$ des zweiten Wechselspannungsgenerators 5. Durch Drücken einer Taste 11 bis 15 wird ein niederfrequenter Differenzfrequenzwert $(\Delta f)_i$ angewählt. Mit diesem niederfrequenten Differenzfrequenzwert $(\Delta f)_i$ wird dann im zweiten Wechselspannungsgenerator 5 die Frequenz $f_1$ zur Bildung der Frequenz $f_2$ amplituden-, phasen- oder frequenzmoduliert. Im Ausführungsbeispiel der Figur 1 sind die Differenzfrequenzwerte $(\Delta f)_i$ entsprechend den eingangs schon erwähnten Werten wie folgt gestaffelt:

$(\Delta f)_1 = 0,1$ Hz bis 1 Hz (gewobbelt)
$(\Delta f)_2 = 2,5$ Hz bis 25 Hz (gewobbelt)
$(\Delta f)_3 = 10$ Hz
$(\Delta f)_4 = 50$ Hz
$(\Delta f)_5 = 200$ Hz

Gemäß der Erfindung ist die Einstellvorrichtung 9 der Intensitätseinstellvorrichtung über eine Signalsteuerleitung 16 mit der Frequenzanwahlvorrichtung 10 verbunden. Die Einstellvorrichtung 9 steuert, sobald sie betätigt wird, die Frequenzanwahlvorrichtung 10 in dem Sinne, daß diese selbsttätig für die Zeitdauer der Intensitätsänderung und eines darauffolgenden Sicherheitsintervalls, z.B. 3sec, dem zweiten Wechselspannungsgenerator 5 über die Verbindungsleitung 17 den jeweils höchsten der vorwählbaren Differenzfrequenzwerte, also im vorliegenden Fall z.B. ( $\Delta$ f)$_5$ = 200 Hz, zuführt. Aufgrund dessen erzeugt also der zweite Wechselspannungsgenerator 5 für die Zeitdauer jeder Intensitätsänderung einschließlich des nachfolgenden 3 sec-Sicherheitsintervalls eine zweite Wechselspannung U$_2$ mit der Frequenz f$_2$ = f$_1$ + 200 Hz = 5,2 kHz (oder f$_2$ = f$_1$ - 200 Hz = 4,8 kHz je nach Wahl des Vorzeichens der Differenzfrequenz ( $\Delta$ f)$_5$. Dadurch bildet sich im Patientenkörper eine Schwebung von 200 Hz. Die Frequenz dieser Schwebung beträgt also ein Vielfaches des Reziprokwertes der Zeit, die eine Bedienungsperson im Normalfall zur Intensitätseinstellung benötigt. Der Intensitätswert wird also immer richtig eingestellt. Schmerzreaktionen beim Patienten aufgrund zu hoher Stromstärken können nicht auftreten.

Während der Zeitdauer der Einstellung des höchsten Differenzfrequenzwertes ( $\Delta$ f)$_5$ = 200 Hz sind alle anderen Differenzfrequenzwerte automatisch gesperrt. Erst nach Ablauf des Sicherheitsintervalls werden alle Differenzfrequenzwerte ( $\Delta$ f)$_i$ wieder zur freien Anwahl freigegeben. Dieser Sachverhalt wird anhand der Figur 2 detaillierter dargestellt.

Gemäß Figur 2 werden die Differenzfrequenzen ( $\Delta$ f)$_1$ bis ( $\Delta$ f)$_5$ von Niederfrequenzgeneratoren 18 bis 22 erzeugt. Die Niederfrequenzgeneratoren 18 und 19 umfassen dabei Wobbelgeneratoren. Ein Differenzfrequenzwert ( $\Delta$ f)$_i$ kann durch Druck eines Tastenschalters 11 bis 15, wodurch ein zugeordneter Schaltkontakt 23 bis 27 (schematische Darstellung) geschlossen wird, angewählt werden. Der angewählte Wert gelangt dann über den geschlossenen Schaltkontakt 23 bis 27 sowie einen normalerweise in Schaltstellung A befindlichen Umschalter 28 auf die Verbindungsleitung 17 zum zweiten Wechselspannungsgenerator 5.

Der Umschalter 28 sowie ein weiterer Einschalter 29 für den Niederfrequenzgenerator 22 sind vom Ausgangssignal S eines Zeitschalters 30 über Schaltleitungen 31 bzw. 32 schaltbar. Solange der Zeitschalter 30 kein Ausgangssignal S liefert, ist der Umschalter 28 in der Schaltstellung A und der weitere Einschalter 29 ist geöffnet. Während der Zeitdauer, in der der Zeitschalter 30 ein Ausgangssignal S erzeugt, ist hingegen (wie in der Figur 2 dargestellt) der Umschalter 28 in der Position B und der weitere Einschalter 29 ist geschlossen. In dieser Situation ist also der 200 Hz-Niederfrequenzgenerator 22, unabhängig davon, ob vorher sein Tastenschalter 15 gedrückt worden war, immer eingeschaltet und das von ihm erzeugte 200 Hz-Signal gelangt über den in der Schaltstellung B befindlichen Schalter 28 zur Verbindungsleitung 17 und damit zum zweiten Wechselspannungsgenerator 5.

Der Zeitschalter 30 wird bei jeder Betätigung der Einstellvorrichtung 9 über die Signalsteuerleitung 16 jeweils immer in dem Sinne gesteuert, daß er ein Ausgangssignal S von der Dauer der Intensitätsänderung (solange also die Betätigung der Einstellvorrichtung andauert) und des sich anschließenden 3 sec-Sicherheitsintervalls erzeugt. Damit wird also, wie erwünscht, gewährleistet, daß immer dann, wenn und so lange, wie die Einstellvorrichtung 9 betätigt wird, der zweite Wechselspannungsgenerator 5 mit dem eingeschalteten und damit aktiven 200 Hz Niederfrequenzgenerator zur Erzeugung einer zweiten Wechselspannung U$_2$ mit (im vorliegenden Fall) der Frequenz f$_1$ = f$_2$ + 200 Hz verbunden ist.

Erst nach Ablauf des 3 sec-Sicherheitsintervalls legt sich der Umschalter 28 wieder in die Schaltstellung A und der weitere Einschalter 29 öffnet sich. Es kann jetzt entweder eine bereits zuvor über einen Tastenschalter 11 bis 15 angewählte Differenzfrequenz ( $\Delta$ f)$_i$ auf die Verbindungsleitung 17 zum zweiten Wechselspannungsgenerator 5 gegeben werden oder es kann durch Drücken eines Tastenschalters erst eine neue Differenzfrequenz eingestellt werden. In der Figur 2 war z.B. vor der Vornahme einer Intensitätsänderung bereits der Tastenschalter 11 gedrückt worden (Schaltkontakt 23 ist geschlossen). Der Niederfrequenzgenerator 18 war also während der Zeitdauer der Intensitätsänderung einschließlich des 3 sec-Sicherheitsintervalls bereits dauernd eingeschaltet. Nach Rückschalten des Umschalters 28 in die Schaltstellung A und Öffnen des weiteren Einschalters 29 erhält der zweite Wechselspannungsgenerator 5 also automatisch die zuvor angewählte Differenzfrequenz ( $\Delta$ f)$_1$.

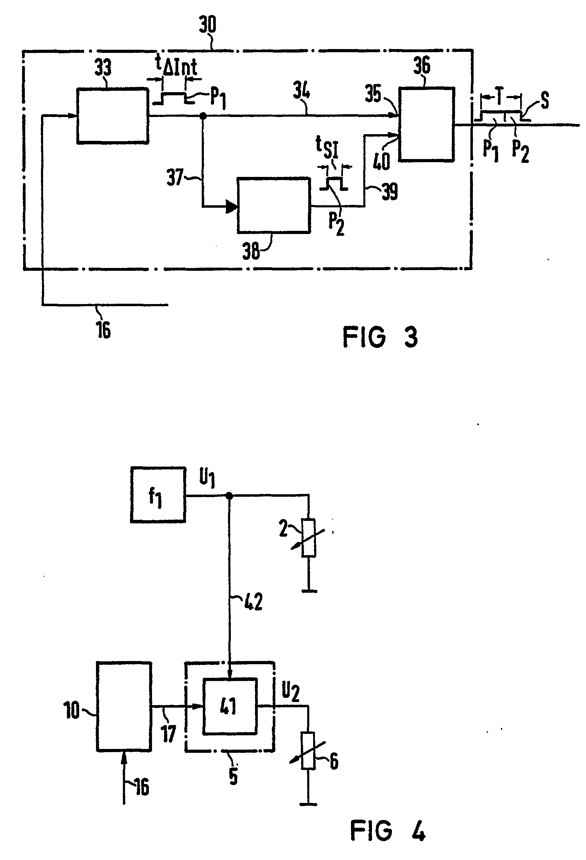

Gemäß der Figur 3 umfaßt der Zeitschalter 30 einen Pulserzeuger 33, der während der Zeitdauer t $\Delta$ Int einer jeden Intensitätsänderung einen Ausgangsimpuls P$_1$ von der Dauer t $\Delta$ Int erzeugt. Dieser Ausgangsimpuls P$_1$ wird einerseits über eine Signalleitung 34 einem ersten Eingang 35 eines ODER-Gliedes 36 und andererseits über eine Signalleitung 37 dem Eingang einer monostabilen Kippstufe 38 zugeleitet. Der Impuls P$_1$ durchläuft das ODER-Glied 36 und erscheint am Ausgang des ODER-Gliedes wieder als Impuls P$_1$. Am Ende des Impulses P$_1$ wird durch die Abfallflanke jedoch die monostabile Kippstufe 38 gesetzt. Die Zeitkonstante der monostabilen Kippstufe 38 beträgt 3 sec. Aus diesem Grunde liefert die monostabile Kippstufe 38 in ihrem Ausgang, der über eine Signalleitung 39 mit einem zweiten Eingang 40 des ODER-Gliedes 36 verbunden ist, einen Impuls P$_2$ mit der Dauer t$_{SI}$ = 3 sec, was der Dauer des Sicherheitsintervalls entspricht. Der Impuls P$_2$ durchläuft ebenfalls das ODER-Glied 36 und verlängert am Ausgang des ODER-Gliedes 36 den Impuls P$_1$ zum gestaffelten Ausgangssignal S = P$_1$ + P$_2$ mit der Gesamtdauer T = t $\Delta$ Int + t$_{SI}$.

Das Prinzipschaltbild der Figur 1 kann gemäß Figur 4 dahingehend modifiziert werden, daß der zweite Wechselspannungsgenerator 5 lediglich ein Modulator 41 ist, der die über eine abgezweigte Signalleitung 42 zugeführte erste Wechselspannung $U_1$ mit der Frequenz $f_1$ mit dem über die Verbindungsleitung 17 zugeführten niederfrequenten Signal ( $\Delta$ f)$_i$ z.B. frequenzmoduliert.

Selbstverständlich können die beiden Wechselspannungsgeneratoren 1 und 5 der Figur 1 aber auch über einen Phasenregelkreis z.B. gemäß DE-PS 26 15 157 miteinander verbunden werden. Das zugeführte niederfrequente Signal ( $\Delta$ f)$_i$ moduliert dann die Frequenz des zweiten Wechselspannungsgenerators in der Phase.

Das Ausführungsbeispiel zeigt ein zweikreisiges System. Selbstverständlich kann dieses System noch um zusätzliche Kreise erweitert werden. Darüber hinaus kann die Intensitätseinstellung z.B. auch digital vorgenommen werden.

## Patentansprüche

1. Interferenzstrom-Therapiegerät mit einem ersten Wechselspannungsgenerator, der eine erste Wechselspannung mit einer ersten Frequenz erzeugt, mit einem zweiten Wechselspannungsgenerator, der in Abhängigkeit von einer Frequenzanwahlvorrichtung eine ausgewählte zweite Wechselspannung aus einer Mehrzahl von in der Frequenz voneinander und zur ersten Frequenz unterschiedlichen Wechselspannungen erzeugt und mit einer Intensitätseinstellvorrichtung zum Einstellen der Intensität der ersten und zweiten Wechselspannung, **dadurch gekennzeichnet**, daß die Intensitätseinstellvorrichtung (2, 3, 6, 7, 9) die Frequenzanwahlvorrichtung (10) steuert in dem Sinne, daß durch sie jeweils bei Betätigung der Intensitätseinstellvorrichtung während der Zeitdauer (t $_{\Delta Int}$) der Intensitätsänderung und eines darauffolgenden Sicherheitsintervalls (t$_{SI}$) der zweite Wechselspannungsgenerator (5) sich selbsttätig auf eine solche Frequenz ($f_2$) der zweiten Wechselspannung ($U_2$) einstellt, die sich von der ersten Frequenz ($f_1$) der ersten Wechselspannung ($U_1$) des ersten Wechselspannungsgenerators (1) um einen Frequenzdifferenzwert (( $\Delta$ f)$_5$) unterscheidet, der wenigstens gleich oder größer ist als der Reziprokwert der Zeit, die eine Bedienungsperson im Normalfall zur Intensitätseinstellung benötigt und daß nach Ablauf des Sicherheitsintervalls der zweite Wechselspannungsgenerator (5) sich selbsttätig auf einen zuvor manuell in Abhängigkeit von der Frequenzanwahlvorrichtung (10) vorgewählten oder noch vorzuwählenden zweiten Frequenzwert ($f_2$ = $f_1$ + ( $\Delta$ f)$_i$) einstellt.

2. Interferenzstrom-Therapiegerät nach Anspruch 1, **dadurch gekennzeichnet**, daß der Differenzfrequenzwert (( $\Delta$ f)$_i$) zwischen der ersten Frequenz ($f_1$) der ersten Wechselspannung ($U_1$) des ersten Wechselspannungsgenerators (1) und der angewählten zweiten Frequenz ($f_2$ = $f_1$ + ( $\Delta$ f)$_i$) der zweiten Wechselspannung ($U_2$) des zweiten Wechselspannungsgenerators (5) durch manuelle Eingabe des erwünschten Differenzfrequenzwertes (( $\Delta$ f)$_i$) an der Anwahlvorrichtung (10) nach Ablauf des Sicherheitsintervalls vorwählbar ist.

3. Interferenzstrom-Therapiegerät nach Anspruch 1 oder 2, **dadurch gekennzeichnet**, daß das Sicherheitsintervall (t$_{SI}$) wenigstens gleich oder größer ist als die Zeit, die der zu behandelnde Patient im Normalfall zur Reaktion auf die geänderte Intensität benötigt.

4. Interferenzstrom-Therapiegerät nach Anspruch 3, **dadurch gekennzeichnet**, daß das Sicherheitsintervall (t$_{SI}$) im Bereich 1 bis 5 sec, vorzugsweise bei 3 sec, liegt.

5. Interferenzstrom-Therapiegerät nach einem der Ansprüche 1 bis 4, **dadurch gekennzeichnet**, daß die zweite Wechselspannung ($U_2$) des zweiten Wechselspannungsgenerators (5) sich während der Zeitdauer (t $_{\Delta Int}$) der Intensitätsänderung und dem darauffolgenden Sicherheitsintervall selbsttätig auf eine solche Frequenz ($f_2$) einstellt, die sich von der ersten Frequenz ($f_1$) der ersten Wechselspannung ($U_1$) des ersten Wechselspannungsgenerators (1) um einen Differenzfrequenzwert $\geq$ 1 Hz unterscheidet.

6. Interferenzstrom-Therapiegerät nach einem der Ansprüche 1 bis 5, **dadurch gekennzeichnet**, daß die Frequenz ($f_2$) der zweiten Wechselspannung ($U_2$) des zweiten Wechselspannungsgenerators (5) sich selbsttätig auf die jeweils höchste der vorwählbaren zweiten Frequenz ($f_2$ = $f_1$ + ( $\Delta$ f)$_5$) einstellt.

## Claims

1. Interference current therapy unit having a first alternating voltage generator which generates a first alternating voltage with a first frequency, a second alternating voltage generator which generates a selected second alternating voltage, as a function of a frequency section arrangement, from a plurality of alternating voltages which, in terms of frequency, differ from each other and from the first frequency and an intensity adjustment arrangement for adjusting the intensity of the first and second alternating voltage, characterised in that the intensity adjustment arrangement (2, 3, 6, 7, 9) controls the frequency selection arrangement (10) in the sense that by it, in each case, in the event of actuation of the intensity adjustment arrangement, during the period (t$_{\Delta Int}$) of the intensity change and a subsequent safety interval (t$_{SI}$) the second alternating voltage generator (5) is automatically adjusted to a frequency ($f_2$) of the second alternating voltage ($U_2$) of such a kind which differs from the first frequency ($f_1$) of the first alternating voltage ($U_1$) of the first alternating voltage generator (1) by a frequency differential value (($\Delta f)_5$) which is at least equal to or greater than the reciprocal value of the time which an operator requires, in the normal case, for the purpose of intensity adjustment and that at the end of the safety interval the second alternating voltage generator (5) is automatically adjusted to a second frequency value ($f_2$ = $f_1$ + ($\Delta f)_i$), which was previously preselected manually as a function of the frequency selection arrangement (10) or which is still to be preselected.

2. Interference current therapy unit according to claim 1, characterised in that the difference frequency value ((Δf)ᵢ) between the first frequency (f₁) of the first alternating voltage (U₁) of the first alternating voltage generator (1) and the selected second frequency (f₂ = f₁ + (Δf)ᵢ) of the second alternating voltage (U₂) of the second alternating voltage generator (5) can be preselected by manual input of the desired difference frequency value ((Δf)ᵢ) at the selection arrangement (10) at the end of the safety interval.

3. Interference current therapy unit according to claim 1 or 2, characterised in that the safety interval (tₛᵢ) is at least equal to or greater than the time which the patient to be treated requires, in the normal case, for the reaction to the changed intensity.

4. Interference current therapy unit according to claim 3, characterised in that the safety interval (tₛᵢ) lies in the range of 1 to 5 sec, preferably at 3 sec.

5. Interference current therapy unit according to one of the claims 1 to 4, characterised in that the second alternating voltage (U₂) of the second alternating voltage generator (5) is automatically adjusted during the period (t_ΔInt) of the intensity change and the subsequent safety interval to a frequency (f₂) of such a kind which differs from the first frequency (f₁) of the first alternating voltage (U₁) of the first alternating voltage generator (1) by a difference frequency value ≥ 1 Hz.

6. Interference current therapy unit according to one of the claims 1 to 5, characterised in that the frequency (f₂) of the second alternating voltage (U₂) of the second alternating voltage generator (5) is automatically adjusted to the highest frequency in each case of the preselectable second frequency (f₂ = f₁ + (Δf)₅).

**Revendications**

1. Appareil de thérapie utilisant un courant interférentiel, comportant un premier générateur de tension alternative qui produit une première tension alternative possédant une première fréquence, un second générateur de tension alternative qui produit, en fonction d'un dispositif de sélection de fréquences, une seconde tension alternative sélectionnée à partir d'une multiplicité de tensions alternatives, dont les fréquences diffèrent entre elles et de la première fréquence, et comportant un dispositif de réglage et seconde tensions alternatives, caractérisé par le fait que le dispositif (2, 3, 6, 7, 9) de réglage de l'intensité commande le dispositif (10) de sélection des fréquences de manière que lors de l'actionnement du dispositif de réglage de l'intensité pendant l'intervalle de temps (t_ΔInt) de la variation d'intensité et d'un intervalle de sécurité suivant (tₛᵢ), le second générateur de tension alternative (5) se règle automatiquement, sur la base dudit dispositif de sélection de fréquence, sur une fréquence (f₂) de la seconde tension alternative (U₂), qui diffère de la première fréquence (f₁) de la première tension alternative (U₁) du premier générateur de tension alternative (1), d'une valeur de différence de fréquence ((Δf)₅), égale ou supérieure à l'inverse de la durée nécessaire à un opérateur pour régler l'intensité, dans le cas normal, et qu'après l'écoulement de l'intervalle de sécurité, le second générateur de tension alternative (5) se règle automatiquement sur une seconde valeur de fréquence (f₂ = f₁ + (Δf)ᵢ), présélectionnée par avance ou pouvant encore être présélectionnée, manuellement, en fonction du dispositif (10) de sélection des fréquences.

2. Appareil de thérapie utilisant un courant interférentiel suivant la revendication 1, caractérisé par le fait que la valeur de la différence de fréquence ((Δf)ᵢ) entre la première fréquence (f₁) de la première tension alternative (U₁) du premier générateur de tension alternative (1) et de la seconde fréquence sélectionnée (f₂ = f₁ + (Δf)ᵢ) de la seconde tension alternative (U₂) du second générateur de tension alternative (5) peut être présélectionnée au moyen d'une introduction manuelle de la valeur désirée de la différence de fréquence ((Δf)ᵢ) dans le dispositif de sélection (10) après l'écoulement de l'intervalle de sécurité.

3. Appareil de thérapie utilisant un courant interférentiel suivant la revendication 1 ou 2, caractérisé en ce que l'intervalle de sécurité (tₛᵢ) est au moins égal ou supérieur à la durée, qui est nécessaire pour que le patient devant être traité réagisse, dans le cas normal, à l'intensité modifiée.

4. Appareil de thérapie à courant interférentiel suivant la revendication 3, caractérisé par le fait que l'intervalle de sécurité (tₛᵢ) se situe dans la plage comprise entre 1 et 5 s, et, de préférence, est égal à 3 s.

5. Appareil de thérapie utilisant un courant interférentiel suivant l'une des revendications 1 à 4, caractérisé par le fait que la seconde tension alternative (U₂) du second générateur de tension alternative (5) se règle automatiquement, pendant la durée (t_ΔInt) de la variation d'intensité et pendant l'intervalle de sécurité suivant, sur une fréquence (f₂), qui diffère de la première fréquence (f₁) de la première tension alternative (U₁) du premier générateur de tension alternative (1), d'une valeur de différence de fréquence ≥ Hz.

6. Appareil de thérapie utilisant un courant interférentiel suivant l'une des revendications 1 à 5, caractérisé par le fait que la fréquence (f₂) de la seconde tension alternative (U₂) du second générateur de tension alternative (5) se règle automatiquement sur la valeur maximale respective de la seconde fréquence pouvant être présélectionnée (f₂ = f₁ + (Δf)₅).

FIG 1

FIG 2

EP 0 241 695 B1

FIG 3

FIG 4